(19) Europäisches Patentamt / European Patent Office / Office européen des brevets

(11) **EP 4 397 680 A1**

(12) **EUROPEAN PATENT APPLICATION**

(43) Date of publication:
**10.07.2024 Bulletin 2024/28**

(21) Application number: **24162117.6**

(22) Date of filing: **29.12.2022**

(51) International Patent Classification (IPC):
**C07K 14/725** (2006.01) **A61K 38/00** (2006.01)

(52) Cooperative Patent Classification (CPC):
**C07K 14/7051; A61K 38/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(62) Document number(s) of the earlier application(s) in accordance with Art. 76 EPC:
**22217237.1 / 4 393 942**

(71) Applicant: **Keshihua (Nanjing) Biotechnology Co., Ltd**
**210031 Nanjing Jiangsu (CN)**

(72) Inventor: **JIAO, Shiping**
**Nanjing Jiangsu 210031 (CN)**

(74) Representative: **ABG Intellectual Property Law, S.L.**
**Avenida de Burgos, 16D**
**Edificio Euromor**
**28036 Madrid (ES)**

Remarks:
•The complete document including Reference Table(s) and the Sequence Listing(s) can be downloaded from the EPO website
•This application was filed on 07.03.2024 as a divisional application to the application mentioned under INID code 62.

(54) **TCR, POLYPEPTIDE, EXPRESSION VECTOR, HOST CELL, PHARMACEUTICAL COMPOSITION AND METHOD FOR OBTAINING TCR**

(57) Disclosed in the present application is a T cell receptor (TCR) capable of specifically recognizes MAGE-A4 antigenic peptide, including a TCRα Polypeptide having at least 90% identity with any one selected from a group consisting of sequences of SEQ ID No: 1 to SEQ ID No: 30 and a TCRβ polypeptide having at least 90% identity with any one selected from a group consisting of sequences of SEQ ID No: 31 to SEQ ID No: 60, in which the TCRα polypeptides are in one-to-one correspondence with the TCRβ polypeptides in order.

EP 4 397 680 A1

**Description**

TECHNICAL FIELD

The present application relates to the technical field of cellular immunotherapy, and, in particular, to a T cell receptor capable of recognizing MAGE-A4 antigen peptide, a polypeptide, an expression vector, a host cell, a pharmaceutical composition and a method for obtaining TCR.

BACKGROUND

A melanoma antigen family A4 (MAGE A4) belongs to a cancer-testis antigen family. It is expressed in a variety of tumor cells, but only in normal tissues of adult testis and placenta. Therefore, MAGE A4 is an ideal tumor treatment target. However, because this target is not a tumor cell surface antigen, it is difficult to specifically recognize the target by using an existing antibody technology and chimeric antigen receptor technology.

A T cell receptor (TCR) is an antibody-like molecule expressed on the surface of T cells. It can be divided into αβ-type TCR and γδ-type TCR according to proteins constituting the receptor. Among them, αβ-type TCR can specifically recognize a short antigenic peptide MHC complex presented by a major histocompatibility complex (MHC; a human MHC is called human leucocyte antigen (HLA)). Therefore, TCR can specifically recognize an intracellular cancer antigen presented to a surface of tumor cells by tumor cell MHC, such as MAGE A4, which can break through a limitation present in existing technologies such as the aforementioned antibody technology and chimeric antigen receptor technology.

TCR has diversity, and this diversity is considered to be one of the important mechanisms of host defense. Diversity is produced due to random recombination, insertion, deletion and replacement of different TCR-αβ gene fragments, which form a polymorphic TCR library. Theoretically, the gene sequence encoding TCR in a genome can create $10^{15}$ to $10^{20}$ TCR clones (one clone is a group of T cells expressing a specific TCR).

At present, in some related technologies, a TCR sequences of a specific target is mainly screened through an in vitro test by those skilled in the art. For example, in some related technologies, TCR sequences of MAGE A4 or MAGE B2 are screened by stimulating naive T cells in vitro to generate a new TCR sequence. Although the specific TCR-T screened by this method can identify its specific target and pass a functional verification (cell killing, mouse tumor eradicating experiment, or the like), T cells obtained by the above method is not subjected to thymus negative selection to remove their autoimmunity, therefore, TCR-T cells thus constructed may attack one's own normal tissues and produce immunotoxicity. For example, in some related technologies, MAGE-A3 TCR-T cells obtained via in vitro screening abnormally recognize cardiac myonectin since they are not subjected to thymus negative screening, resulting in intolerable cardiotoxicity in clinical trials.

In addition, at present, sarcoma is primarily selected as an indication for clinical development and research in clinical applications in TCR-T technologies for MAGE A4, but this indication accounts for an extremely low proportion in tumor patients in China.

BRIEF SUMMARY

In view of this, the present application provides a natural T cell receptor (TCR) that specifically recognizes MAGE-A4 positive tumors, a TCR expression vector and a host cell, for the treatment of tumor diseases.

In a first aspect, the present application provides a T cell receptor (TCR) that can specifically recognizes MAGE-A4 antigenic peptide, which includes a TCRα Polypeptide having at least 90% identity with any one selected from a group consisting of sequences of SEQ ID No: 1 to SEQ ID No: 30 and a TCRβ polypeptide having at least 90% identity with any one selected from a group consisting of sequences of SEQ ID No: 31 to SEQ ID No: 60, in which the TCRα polypeptides are in one-to-one correspondence with the TCRβ polypeptides in order.

In some embodiments, the antigenic peptide is HLA-A2 restricted. For example, in some further embodiments of the present application, the HLA-A2 is HLA-A*0201 typing.

In some embodiments, sequences of SEQ ID No: 1 to SEQ ID No: 30 sequentially contain a sequence having at least 95% identity with CDRα1 (SEQ ID No: 61 to SEQ ID No: 90), CDRα2 (SEQ ID No: 91 to SEQ ID No: 120) and CDRα3 (SEQ ID No: 121 to SEQ ID No: 150), in which the CDRα1, the CDRα2 and the CDRα3 are in one-to-one correspondence with each other in order; and sequences of SEQ ID No: 31 to SEQ ID No: 60 sequentially contain a sequence having at least 95% identity with CDRβ1 (SEQ ID No: 151 to SEQ ID No: 180), CDRβ2 (SEQ ID No: 181 to SEQ ID No: 210) and CDRβ3 (SEQ ID No: 211 to SEQ ID No: 240), in which the CDRβ1, the CDRβ2 and the CDRβ3 are in one-to-one correspondence with one another in order. For example, TCRα polypeptide of SEQ ID No: 1 contains a sequence having at least 95% identity with CDRα1 of SEQ ID No: 61, a sequence having at least 95% identity with CDRα2 of ID No: 91, and a sequence having at least 95% identity with CDRα3 of SEQ ID No: 121, and so on. For another example, TCRβ polypeptide of SEQ ID No: 31 contains a sequence having at least 95% identity with CDRβ1 of SEQ ID No: 151, a sequence having at least 95% identity with CDRβ2 of SEQ ID No: 181, and a sequence having at least 95% identity with CDRβ3 of SEQ ID No: 211, and so on.

In some further embodiments, the sequences of SEQ ID No: 1 to SEQ ID No: 30 sequentially contain a sequence having at least 99% identity with CDRα1 (SEQ ID No: 61 to SEQ ID No: 90), CDRα2 (SEQ ID No: 91 to SEQ ID No: 120) and CDRα3 (SEQ ID No: 121 to SEQ ID No: 150), in which the CDRα1, the CDRα2 and the CDRα3 are in one-to-one correspondence with each other in order; and the sequences of SEQ ID No: 31 to SEQ ID No: 60 sequentially contain a sequence having at least 95% identity with CDRβ1 (SEQ ID No: 151 to SEQ ID No: 180), CDRβ2 (SEQ ID No: 181 to SEQ ID No: 210) and CDRβ3 (SEQ ID No: 211 to SEQ ID No: 240), in which the CDRβ1, the CDRβ2 and the CDRβ3 are in one-to-one correspondence with each other in order. For example, TCRα polypeptide of SEQ ID No: 1 contains a sequence having at least 99% identity with CDRα1 of SEQ ID No: 61, a sequence having at least 99% identity with CDRα2 of ID No: 91, and a sequence having at least 99% identity with CDRα3 of SEQ ID No: 121, and so on. For another example, TCRβ polypeptide of SEQ ID No: 31 contains a sequence having at least 99% identity with CDRβ1 of SEQ ID No: 151, a sequence having at least 99% identity with CDRβ2 of SEQ ID No: 181, and a sequence having at least 99% identity with CDRβ3 of SEQ ID No: 211, and so on.

In some embodiments, the sequences of SEQ ID No: 1 to SEQ ID No: 30 sequentially contain CDRα1 (SEQ ID No: 61 to SEQ ID No: 90), CDRα2 (SEQ ID No: 91 to SEQ ID No: 120) and CDRα3 (SEQ ID No: 121 to SEQ ID No: 150), in which the CDRα1, the CDRα2 and the CDRα3 are in one-to-one correspondence with each other in order; and the sequences of SEQ ID No: 31 to SEQ ID No: 60 sequentially contain CDRβ1 (SEQ ID No: 151 to SEQ ID No: 180), CDRβ2 (SEQ ID No: 181 to SEQ ID No: 210) and CDRβ3 (SEQ ID No: 211 to SEQ ID No: 240), in which the CDRβ1, the CDRβ2 and the CDRβ3 are in one-to-one correspondence with each other in order. For example, TCRα polypeptide of SEQ ID No: 1 contains CDRα1 of SEQ ID No: 61, CDRα2 of ID No: 91, and CDRα3 of SEQ ID No: 121, and so on. For another example, TCRβ polypeptide of SEQ ID No: 31 contains CDRβ1 of SEQ ID No: 151, CDRβ2 of SEQ ID No: 181, and CDRβ3 of SEQ ID No: 211, and so on.

In some embodiments, the TCRα polypeptide has at least 95% identity with any one selected from a group consisting of sequences of SEQ ID No: 1 to SEQ ID No: 31, and the TCR β polypeptide has at least 95% identity with any one selected from a group consisting of sequences of SEQ ID No: 32 to SEQ ID No: 62, in which all of the TCRα polypeptides are in one-to-one correspondence with all of the TCRβ polypeptides in order.

In the present application, the TCRα polypeptide has at least 99% identity with any one selected from a group consisting of sequences of SEQ ID No: 1 to SEQ ID No: 31, and the TCRβ polypeptide has at least 99% identity with any one selected from a group consisting of sequences of SEQ ID No: 32 to SEQ ID No: 62, in which all of the TCRα polypeptides are in one-to-one correspondence with all of the TCRβ polypeptides in order.

In some embodiments, a T cell receptor includes, or consists of, one of SEQ ID Nos. 1-30, one of SEQ ID Nos. 243, 245 and 247, one of SEQ ID Nos. 241 and 242, one of SEQ ID Nos. 31-60, and one of SEQ ID Nos. 244, 246 and 248, in which all of the SEQ ID Nos. 1-30 are in one-to-one correspondence with all of the SEQ ID Nos. 31-60 in order. For example, in one particular embodiment, the T cell receptor includes, or consists of, SEQ ID No. 1, SEQ ID No. 245, SEQ ID No. 241, SEQ ID No. 31, and SEQ ID No. 246.

In a second aspect, the present application provides a polypeptide including TCRα polypeptide having sequences CDRα1 (SEQ ID No: 61 to SEQ ID No: 90), CDRα2 (SEQ ID No: 91 to SEQ ID No: 120) and CDRα3 (SEQ ID No: 121 to SEQ ID No: 150) and TCRβ polypeptide having sequences CDRβ1 (SEQ ID No: 151 to SEQ ID No: 180), CDRβ2 (SEQ ID No: 181 to SEQ ID No: 210) and CDRβ3 (SEQ ID No: 211 to SEQ ID No: 240) .

In some embodiments, the polypeptide includes a TCRα polypeptide having at least 90% identity with any one selected from a group consisting of amino acid sequences of SEQ ID No: 1 to SEQ ID No: 30 and a TCRβ polypeptide having at least 90% identity with any one selected from a group consisting of amino acid sequences of SEQ ID No: 31 to SEQ ID No: 60.

In some embodiments, the polypeptide includes a TCRα polypeptide having at least 95% identity with any one selected from a group consisting of amino acid sequences of SEQ ID No: 1 to SEQ ID No: 30 and a TCRβ polypeptide having at least 95% identity with any one selected from a group consisting of amino acid sequences of SEQ ID No: 31 to SEQ ID No: 60.

In some embodiments, the polypeptide includes a TCRα polypeptide of any one selected from a group consisting of sequences of SEQ ID No: 1 to SEQ ID No: 30.

In some embodiments, the polypeptide includes a TCRβ polypeptide of any one selected from a group consisting of sequences of SEQ ID No: 31 to SEQ ID No: 60.

In a third aspect, the present application further provides a polynucleotide capable of encoding a polypeptide according to the second aspect.

In a fourth aspect, the present application further provides an expression vector containing the TCR according to the first aspect. In some embodiments, the expression vector is a viral vector. In some embodiments, the expression vector is a retroviral vector or a lentiviral vector.

In some embodiments, the vector further includes a humanized C domain or a murine C domain or a modified humanized C domain. Further, the humanized C domain contains a humanized C subdomain linking an α chain (sequence SEQ ID No: 243) and a humanized C subdomain linking β chain (sequence SEQ ID No: 244); the murine C domain contains a murine C subdomain linking α chain (sequence SEQ ID No: 245) and a murine C subdomain linking β chain (sequence SEQ ID No: 246); and the modified humanized C domain contains a modified humanized C subdomain linking α chain (sequence SEQ ID No: 247) and a modified humanized C subdomain linking β chain (sequence SEQ ID No: 248).

In some embodiments, the expression vector further includes a linker domain, which is located between TCRα polypeptides and TCRβ polypeptides. In some embodiments, the linker domain is selected from a group consisting of sequences of SEQ ID No: 241 or SEQ ID No: 242.

In some embodiments, in the expression vector, a TCRα chain and a TCRβ chain is connected as follow: TCRα chain V(D) j + humanized C subdomain linking α chain (sequence SEQ ID No: 243) or murine C domain linking α chain (sequence SEQ ID No: 245) or modified humanized C subdomain linking α chain (sequence SEQ ID No: 247) + linker domain (sequence SEQ ID No: 249 or SEQ ID No: 250) + TCRβ chain V(D)J+ humanized C subdomain linking β chain (sequence SEQ ID No: 244) or murine C subdomain linking β chain (sequence SEQ ID No: 246) or modified humanized C subdomain linking β chain (sequence SEQ ID No: 248).

Among them, in a same complete TCR chain, C subdomain linked behind the TCRα chain and C subdomain linked behind the TCRβ chain are of a same source, for example, both are humanized C domain, or both are murine C domain, or both are modified humanized C domain.

In a fifth aspect, the present application further provides a host cell for transforming TCR prepared in the application. In some embodiments, the cells are immune cells.

Further, the immune cells are selected from a group consisting of T cells, NK cells, NKT cells, constant NK cells or other lymphocytes in peripheral blood.

In some embodiments, the host cells are autologous or allogeneic cells isolated from umbilical cord or blood.

In a sixth aspect, the present application further provides a pharmaceutical composition including MAGE-A4 TCR specific cells according to the fifth aspect. In some embodiments, the pharmaceutical composition is used to treat a cancer. In some further embodiments, the cancer is selected from a group consisting of esophageal cancer, gastric cancer, bladder cancer, head and neck tumor or sarcoma.

In a seventh aspect, the present application provides a method for obtaining the TCR according to the first aspect. In some embodiments, the method includes the following steps: (1) Obtaining human MAGE-A4 positive (HLA-A*02:01) tumor tissue by immunohistochemistry and HLA sequence-based typing as potential TCR screening objects, in which the objects are T cells screened by thymus autoimmune tolerance; (2) Preparing a HLA-A*02:01 tetramer containing MAGE-A4 peptide; (3) Digesting the obtained tumor tissue into a single cell suspension, and screening T cells capable of recognizing MAGE-A4 peptide by using HLA-A*02:01 tetramer containing MAGE-A4 peptide prepared in step (2); and (4) Sequencing the T cells screened in step (3) to obtain a TCR V(d)J full-length sequence capable of recognizing MAGE-A4 peptide by single cell multi-omics.

Further, in step (1), the human MAGE-A4 positive (HLA-A*02:01) tumor tissue is obtained by the steps of obtaining surgical or puncture specimens of a MAGE-A4 positive tumor, cutting sections from the specimens and pasting on slides; baking and dewaxing the slides; performing antigen retrieval on the specimens; performing endogenous peroxidase and nonspecific binding protein blocking, diluting MAGE-A4 primary antibody, incubating at 4°C overnight, incubating an enzyme-labeled MAGE-A4 secondary antibody, performing diaminobenzidine (DAB) color development, counterstaining with Harris hematoxylin staining solution, sealing with a neutral gum, and scanning; and confirming HLA-A*02:01 typing.

Further, confirming HLA-A*02:01 typing can be performed by two methods:

Method 1: aligning original expression data obtained from single cell sequencing against a reference genome GRCh38 through a count module of CellRanger to generate an aligned bam file "sorted_genome_bam. BAM" and a cell-gene expression matrix; performing single cell HLA typing based on the bam file and a cell barcode file on a software scHLAacount to generate a file containing all HLA typings detected in the samples, a depth statistics file of individual HLA typings, and a cell-HLA typing depth matrix file for confirming the typing of the samples; or

Method 2: thawing PBMC samples freeze stored in cell cryopreservation solution, washing the sample twice with PBS, centrifuging to remove the cell cryopreservation solution and obtain cell precipitate, extracting RNA by using Qiagen total RNA extraction kit from the cell precipitate, denaturing the RNA, reversely transcribing the RNA by using Vazyme HiScript to obtain cDNA, perform target enrichment on the cDNA by using Abclonal Gloria HS PCR Kit and primers corresponding to HAL1 and HLA2, respectively, to obtain enriched products, subjecting the enriched products to fragmentation, terminal repair, linker ligation, Index PCR amplification, construction and sequencing, and confirming the typing of the samples.

Further, in step (2), MAGE-A4 HLA-A*02:01 tetramer is prepared by the steps of selecting an antigen peptide containing MAGE-A4 and combined with HLA-A*02:01; performing peptide exchange; and preparing the tetramer.

Further, performing the peptide exchange includes:

mixing a peptide stock solution with PBS to obtain a diluted peptide solution, and stored on ice;
adding the diluted peptide and peptide flex-t ™ HLA-A * 02:01 monomer UVX (280004; Biolegend) into a well plate and mixing;
sealing the well plate with a seal and centrifuging to obtain a supernatant;
removing the seal, placing the well plate on ice and irradiating with a UV lamp; and
sealing the well plate, incubating in the dark to collect a liquid as obtained peptide exchange monomer.

Still further, the tetramer is prepared by the steps of:

transferring the peptide exchange monomer to a micro centrifuge tube or a new plate, adding PE streptavidin, mixing, and incubating in the dark on ice;
adding MD-biotin and $NaN_3$ to PBS to obtain a blocking solution, and adding the blocking solution to the well plate after incubation to stop a reaction;
incubating sealed well plate at 2-8 °C overnight;
preparing a target group:
centrifuging the obtained tetramer and stored on ice in the dark;
adjusting a volume with cell staining buffer and culturing on ice;
washing cells with staining buffer, and culturing the cells in the staining buffer; and
collecting samples by flow cytometry.

Further, step (3) is performed as follows: removing connective tissues attached to a surface the obtained tumor tissue, cutting into pieces, shredding, placing into a centrifuge tube, and adding a digestive solution from Miltenyi gentleMACS discociator KIT to digest the tissues; and inspecting quality of cells by using Bio-Rad TC20 automatic cell counter with a microscope.

Further, in step (4), the T cells screened in step (3) are sequenced by single-cell omics to obtain the T cells TCR V(d)J full-length sequence capable of recognizing MAGE-A4 peptide. In particular, the T cells TCR V(d)J full-length sequence capable of recognizing MAGE-A4 peptide is obtained from a tumor by single-cell immunohistochemical sequencing.

The present application further provides a plasmid virus and cell constructed from TCR-T, including the following steps:

(1) constructing of plasmid;
(2) virus packaging
(3) thawing, stimulating, and activating of PBMC;
(4) lentivirus transducting; and
(5) expanding and culturing of cells.

A use of a composition containing an effective therapeutic dose of MAGE-A4 TCR specific cells for the treatment of a cancer is further provided in the present application.

The present application can achieve at least one of the following beneficial effects.

1. In this application, T cells are obtained from tumor tissues of patients with an esophageal cancer, a bladder cancer or a laryngeal cancer with MAGE A4+ and HLA-A02+, and natural TCRs that can recognize MAGE A4+ are screened therefrom. The TCRs obtained by this method are those subjected to the patient's own thymus negative selection, which excludes the TCRs that can react with normal human tissues, and greatly reduces immunotoxicity due to anti autoantigen from the source.

2. In the present application, the expression amount of MAGE A4 is determined in 20 kinds of cancers by the immunohistochemical method, and the results reveal that the target is highly expressed and positive in esophageal cancer, gastric cancer, bladder cancer and laryngeal cancer of cancer patients in China. Considering that an incidence rate of the above cancers in China is higher than that of sarcomas, TCR sequence targeting esophageal cancer, gastric cancer, bladder cancer, laryngeal cancer and sarcomas, an expression vector, an excipient, cells and preparations containing the TCR sequence, and a clinical application of a product containing the same in cancer patients are developed in the present application.

## BRIEF DESCRIPTION OF DRAWINGS

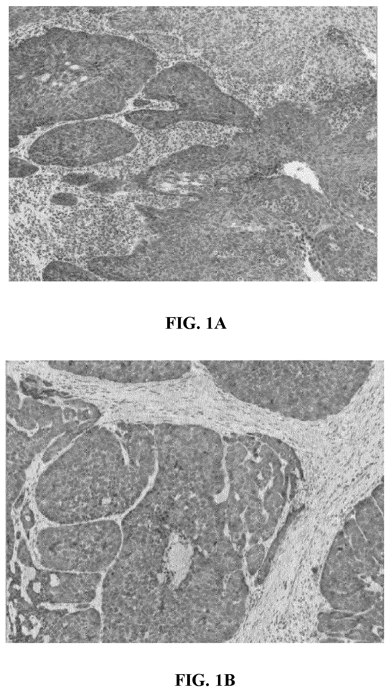

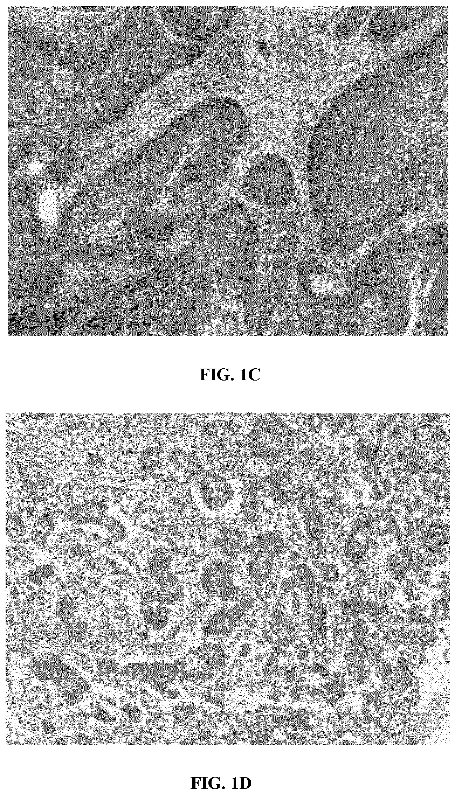

FIGs 1A to 1D show the scanning and screening results of MAGE-A4 positive tumor sections. Among them, FIG. 1A shows the scanning results of of PA001 esophagus moderately differentiated squamous cell carcinoma in clinical phase IA; FIG. 1b shows the scanning results of PA002 esophagus basaloid squamous cell carcinoma with medium to low differentiation in clinical phase IIIB; FIG. 1C shows the scanning results of PA003 glottic moderately differentiated squamous cell carcinoma in clinical phase III; and FIG. 1D shows the scanning results of PA004 bladder invasive urothelial carcinoma in clinical phase II.

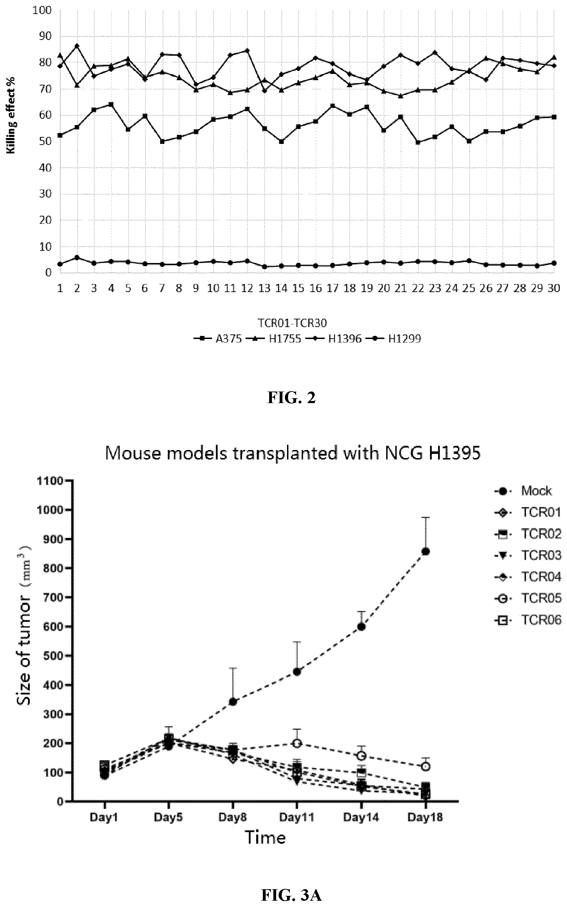

FIG. 2 is a diagram showing a killing effect of a cell line.

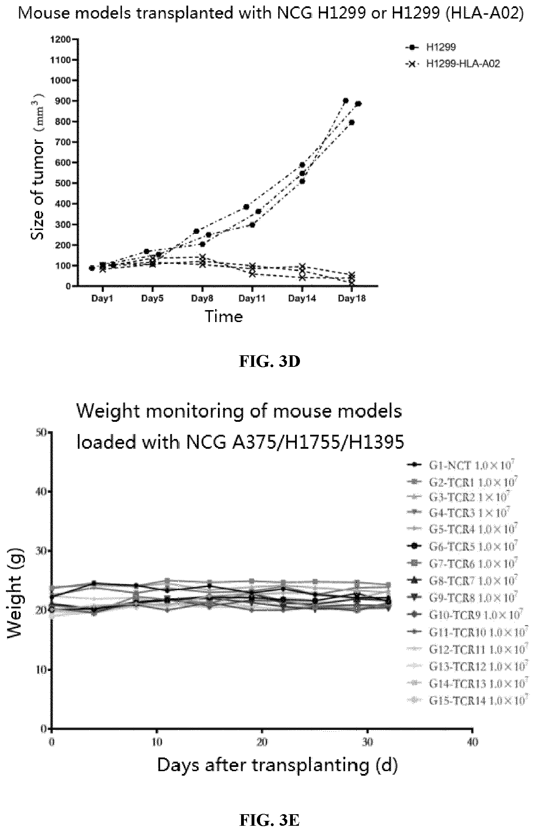

FIG.s 3A to 3D are diagrams showing tumor clearance effect in an experiment, and FIG. 3E is a diagram showing the change in weight of experimental mice.

FIG.s 4A to 4C are diagrams show results of IFN ELISA experiments.

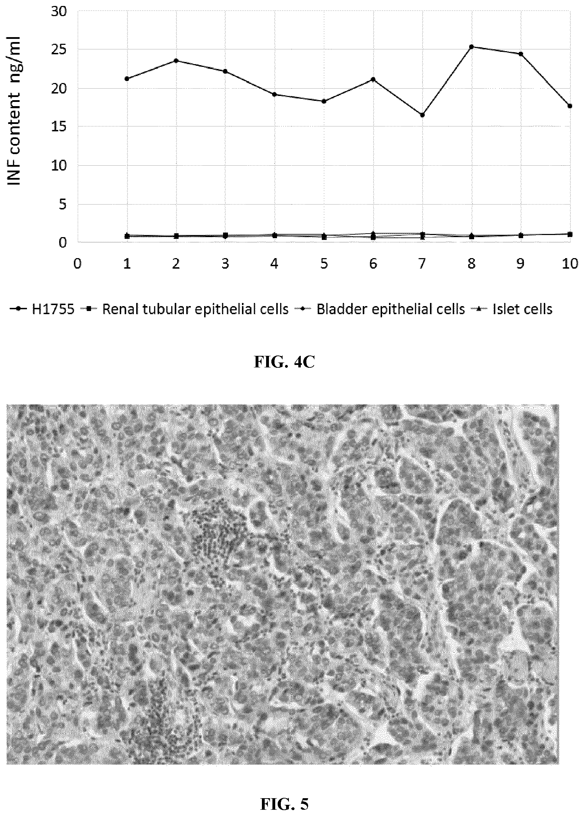

FIG. 5 shows 6PA2021-TCR01 MAGE-A4 IHC pathological staining results (40X) in Example 6.

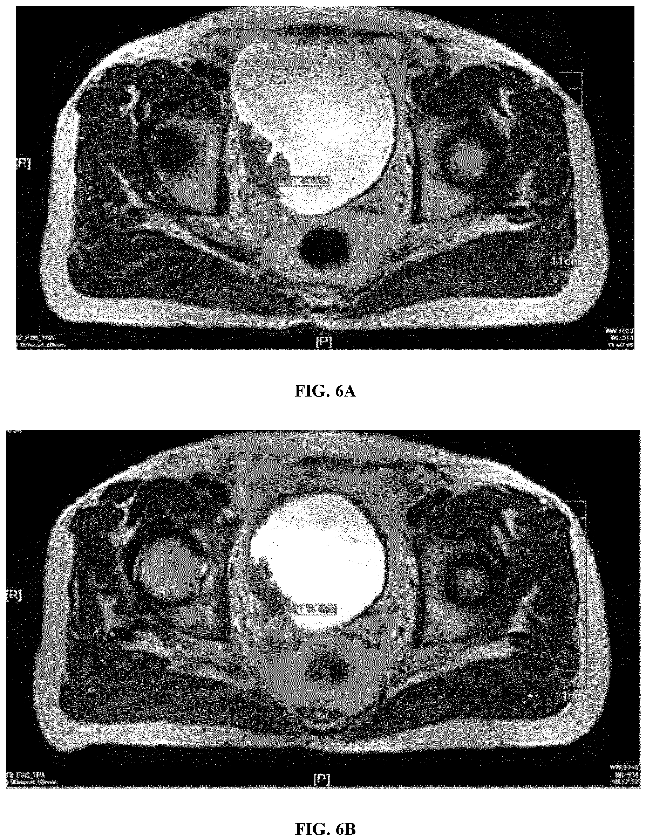

FIG.s 6A-6B are pictures showing a therapeutic effect of TCR-T cells screened according to an embodiment on tumor patients, in which FIG. 6A shows a MR result of intravenously infusing TCR01-05 cell preparation ($5e^{10}$ TCR-T cells) on day 0, and FIG. 6B shows a MR result on day 45, that is, 45 days after intravenous infusion.

Sequence listing

SEQ ID NO:1-SEQ ID NO:30: sequences of TCRα polypeptide chain
SEQ ID NO:31-SEQ ID NO:60: sequences of TCRβ polypeptide chain
SEQ ID NO:61-SEQ ID NO90: sequences of CDRα1 of TCRα polypeptide chain
SEQ ID NO:91-SEQ ID NO120: sequences of CDRα2 of TCRα polypeptide chain
SEQ ID NO: 121-SEQ ID NO: 150: sequences of CDRα3 of TCRα polypeptide chain
SEQ I D NO:151-SEQ ID NO: 180: sequences of CDRβ1 of TCRβ polypeptide chain
SEQ ID NO:181-SEQ ID NO:210: sequences of CDRβ2 of TCRβ polypeptide chain
SEQ ID NO:211-SEQ ID NO:240: sequences of CDRβ3 of TCRβ polypeptide chain
SEQ ID NO:241-SEQ ID NO:242: Joint domain
SEQ ID NO:243: humanized C domain linking α chain
SEQ ID NO:244: humanized C domain linking β chain
SEQ ID NO:245: murine C domain linking α chain
SEQ ID NO:246: murine C domain linking β chain
SEQ ID NO:247: modified humanized C domain linking α chain
SEQ ID NO:248: modified humanized C domain linking β chain

## DETAILED DESCRIPTION

In view of the problems present in existing technologies, the present inventor conducted a lot of research, and found a new technical path, which includes selecting a tumor tissue of a patient with esophageal cancer, bladder cancer or laryngeal cancer showing MAGE A4+ and HLA-A02+, obtaining T cells therefrom, and screening a natural TCR sequence that can recognize MAGE A4+. The TCR obtained by this method is a TCR subjected to the patient's own thymus negative selection, which excludes the TCRs that can react with a normal human tissue, and greatly reduces an off-target toxicity from a source.

In addition, in some related technologies, the TCR-T technology for MAGE A4 primarily selects sarcoma as an indication for clinical development and research in practice, but a proportion of this indication in tumor patients in China is very low. In order to expand the application scope of this technology, the present inventor measured the expression of MAGE A4 in 20 kinds of cancer by immunohistochemical method, and found that the target was highly expressed and positive in esophageal cancer, gastric cancer, bladder cancer and laryngeal cancer of tumor patients in China. Considering that an incidence rate of the above cancers in China is higher than that of sarcomas, a TCR sequence targeting esophageal cancer, gastric cancer, bladder cancer, laryngeal cancer and sarcomas, an expression vector, an excipient, cells and preparations containing the TCR sequence, and a clinical application of a product containing the same in cancer patients are developed in the present application.

The present application is made based on the above discovery.

Unless clearly indicated otherwise in the context, nouns without quantifier modification used in the present application represent one or more, for example, the reference to "cell" includes a plurality of such cells, and the reference to "peptide" includes one or more peptides and their equivalents (for example, polypeptides) known to those skilled in the art.

Additional aspects:

1. A T cell receptor (TCR) capable of specifically recognizing MAGE-A4 antigenic peptide, comprising a TCRα Polypeptide having at least 90% identity with any one selected from a group consisting of sequences of SEQ ID No: 1 to SEQ ID No: 30 and a TCRβ polypeptide having at least 90% identity with any one selected from a group consisting of sequences of SEQ ID No: 31 to SEQ ID No: 60, and the TCRα polypeptides are in one-to-one correspondence with the TCRβ polypeptides in order.

2. The TCR according to aspect 1, wherein the antigenic peptide is HLA-A2 restricted.

3. The TCR according to aspect 2, wherein the HLA-A2 is HLA-A*0201 typing.

4. The TCR according to aspect 1, wherein sequences of SEQ ID No: 1 to SEQ ID No: 30 sequentially contain a sequence having at least 95% identity with CDRα1 (SEQ ID No: 61 to SEQ ID No: 90), CDRα2 (SEQ ID No: 91 to SEQ ID No: 120) and CDRα3 (SEQ ID No: 121 to SEQ ID No: 150), and the CDRα1, the CDRα2 and the CDRα3 are in one-to-one correspondence with each other in order; and sequences of SEQ ID No: 31 to SEQ ID No: 60 sequentially contain a sequence having at least 95% identity with CDRβ1 (SEQ ID No: 151 to SEQ ID No: 180), CDRβ2 (SEQ ID No: 181 to SEQ ID No: 210) and CDRβ3 (SEQ ID No: 211 to SEQ ID No: 240), and the CDRβ1, the CDRβ2 and the CDRβ3 are in one-to-one correspondence with one another in order.

5. The TCR according to aspect 1, wherein sequences of SEQ ID No: 1 to SEQ ID No: 30 sequentially contain a sequence having at least 99% identity with CDRα1 (SEQ ID No: 61 to SEQ ID No: 90), CDRα2 (SEQ ID No: 91 to SEQ ID No: 120) and CDRα3 (SEQ ID No: 121 to SEQ ID No: 150), and the CDRα1, the CDRα2 and the CDRα3 are in one-to-one correspondence with each other in order; and sequences of SEQ ID No: 31 to SEQ ID No: 60 sequentially contain a sequence having at least 95% identity with CDRβ1 (SEQ ID No: 151 to SEQ ID No: 180), CDRβ2 (SEQ ID No: 181 to SEQ ID No: 210) and CDRβ3 (SEQ ID No: 211 to SEQ ID No: 240), and the CDRβ1, the CDRβ2 and the CDRβ3 are in one-to-one correspondence with each other in order.

6. The TCR according to aspect 1, wherein sequences of SEQ ID No: 1 to SEQ ID No: 30 sequentially contain CDRα1 (SEQ ID No: 61 to SEQ ID No: 90), CDRα2 (SEQ ID No: 91 to SEQ ID No: 120) and CDRα3 (SEQ ID No: 121 to SEQ ID No: 150), and the CDRα1, the CDRα2 and the CDRα3 are in one-to-one correspondence with each other in order; and the sequences of SEQ ID No: 31 to SEQ ID No: 60 sequentially contain CDRβ1 (SEQ ID No: 151 to SEQ ID No: 180), CDRβ2 (SEQ ID No: 181 to SEQ ID No: 210) and CDRβ3 (SEQ ID No: 211 to SEQ ID No: 240), and the CDRβ1, the CDRβ2 and the CDRβ3 are in one-to-one correspondence with each other in order.

7. The TCR according to aspect 1, wherein the TCRα polypeptide has at least 95% identity with any one selected from a group consisting of sequences of SEQ ID No: 1 to SEQ ID No:30, and the TCR β polypeptide has at least 95% identity with any one selected from a group consisting of sequences of SEQ ID No: 31 to SEQ ID No: 60, and all of the TCRα polypeptides are in one-to-one correspondence with all of the TCRβ polypeptides in order.

8. The TCR according to aspect 1, wherein the TCRα polypeptide has at least 99% identity with any one selected from a group consisting of sequences of SEQ ID No: 1 to SEQ ID No: 30, and the TCRβ polypeptide has at least 99% identity with any one selected from a group consisting of sequences of SEQ ID No: 31 to SEQ ID No: 60, and all of the TCRα polypeptides are in one-to-one correspondence with all of the TCRβ polypeptides in order.

9. A polypeptide comprising TCRα polypeptide having CDRα1 selected from a group consisting of SEQ ID No: 61 to SEQ ID No: 90, CDRα2 selected from a group consisting of SEQ ID No: 91 to SEQ ID No: 120 and CDRα3 selected from a group consisting of SEQ ID No: 121 to SEQ ID No: 150 and TCRβ polypeptide having CDRβ1 selected from a group consisting of SEQ ID No: 151 to SEQ ID No: 180, CDRβ2 selected from a group consisting of SEQ ID No: 181 to SEQ ID No: 210 and CDRβ3 selected from a group consisting of SEQ ID No: 211 to SEQ ID No: 240.

10. The polypeptide according to aspect 9, wherein the polypeptide comprises a TCRα polypeptide having at least 90% identity with any one selected from a group consisting of amino acid sequences of SEQ ID No: 1 to SEQ ID No: 30 and a TCRβ polypeptide having at least 90% identity with any one selected from a group consisting of amino acid sequences of SEQ ID No: 31 to SEQ ID No: 60.

11. The polypeptide according to aspect 9, wherein the polypeptide comprises a TCRα polypeptide having at least 95% identity with any one selected from a group consisting of amino acid sequences of SEQ ID No: 1 to SEQ ID No: 30 and a TCRβ polypeptide having at least 95% identity with any one selected from a group consisting of amino acid sequences of SEQ ID No: 31 to SEQ ID No: 60.

12. The polypeptide according to aspect 9, wherein the polypeptide comprises a TCRα polypeptide of any one selected from a group consisting of sequences of SEQ ID No: 1 to SEQ ID No: 30.

13 The polypeptide according to aspect 9, wherein the polypeptide includes a TCRβ polypeptide of any one selected from a group consisting of sequences of SEQ ID No: 31 to SEQ ID No: 60.

14. A polynucleotide capable of encoding a polypeptide according to aspect 9.

15. An expression vector containing the TCR according to aspect 1.

**Example 1**

Screening T cells potentially capable of recognizing MAGE-A4 positive solid tumors (HLA-A*02:01 positive patients) by using MAGE-A4 antigen peptide tetramer included the following steps.

(1) Human MAGE-A4 positive (HLA-A*02:01) tumor surgical specimens or puncture tumor tissues were obtained by immunohistochemistry and HLA sequencing typing screening.

In this example, 2 esophageal cancer tissues showing MAGE-A4+, 1 bladder cancer showing MAGE-A4+ and 1 head and neck tumor showing MAGE-A4+ were obtained, and a strong positive expression of MAGE-A4 was confirmed by immunohistochemical method.

The specific methods were as follows:

4 $\mu$m-thick sections were cut from a FFPE specimen, attached on glass slides, baked and dewaxed. Antigen repair was performed on these sections.

After blocking endogenous peroxidase and nonspecific binding protein (goat serum blocking solution), MAGE-A4 primary antibody was diluted by a 1:200, and incubated at 4 °C overnight. An enzyme-labeled secondary antibody was incubated for the next day, subjected to DAB color development, counterstained with Harris hematoxylin staining solution, and finally sealed with a neutral gum. Scanning was performed on Vectra3 multispectral scanner under 40 $\times$ equivalent objective lens. The scanning results are shown in FIGs. 1A-1D. After scanning, the images were analyzed with inForm software to confirm results of screening.

Then HLA-A*02:01 typing was confirmed. In particular, the HLA-A*02:01 typing was confirmed as follows.

Original expression data obtained by single cell sequencing was aligned to a reference genome GRCh38 via a count module of CellRanger (v6.0.2) to generate a BAM file "sorted_genome_bam. BAM" and a cell-gene expression matrix.

Based on the BAM file and a cell barcode file generated by alignment, a software scHLAcount was used to perform HLA typing of single cells, which generated a file of all HLA typing types detected in the sample (labels. tsv), a depth statistics file of individual HLA typing of the specimens (summary.tsv), and a depth matrix file of cell-HLA typing (count_matrix. MTX), for confirming the typing of the specimens.

The HLA typing of 4 patients measured in this Example is shown in Table 1-Table 4.

Table 1

| PA001 HLA Type | Read_counts | HLA_cell_number | Total_cell_num | Pos_Cell_ratio |
|---|---|---|---|---|
| **A*02:01:176** | **123094** | **11594** | **12342** | **0.9394** |
| B*38:01:01:06 | 116695 | 11248 | 12342 | 0.9114 |
| C*12:227 | 107872 | 11254 | 12342 | 0.9118 |

Table 2

| PA002 HLA Type | Read_counts | HLA_cell_number | Total_cell_num | Pos_Cell_ratio |
|---|---|---|---|---|
| **A*02:01:175** | **175081** | **9859** | **10452** | **0.9433** |
| B*54:01:09 | 69756 | 9170 | 10452 | 0.8773 |
| C*01:03:01 | 81025 | 9388 | 10452 | 0.8982 |

Table 3

| PA003 HLA Type | Read_counts | HLA_cell_number | Total_cell_num | Pos_Cell_ratio |
|---|---|---|---|---|
| **A\*02:01:176** | **84405** | **5362** | **5886** | **0.911** |
| B\*08:18 | 84061 | 5065 | 5886 | 0.8605 |
| C\*01:17 | 57872 | 5181 | 5886 | 0.8802 |

Table 4

| PA004 HLA Type | Read_counts | HLA_cell_number | Total_cell_num | Pos_Cell_ratio |
|---|---|---|---|---|
| **A\*02:01:175** | **28510** | **2703** | **2983** | **0.9061** |
| B\*08:18 | 31832 | 2671 | 2983 | 0.8954 |
| C\*03:47 | 2300 | 1287 | 2983 | 0.4314 |
| C\*07:429 | 10576 | 2289 | 2983 | 0.7673 |

(2) Preparation of tetramer containing MAGE-A4 peptide HLA-A*02:01

step S1. 10 mM MAGE-A4 peptide stock solution was selected, which has the following amino acid sequence:

1) KVLEHVVRV (SEQ ID NO:249); 2) GVYDGREHTV (SEQ ID NO:250); 3) ALLEEEEGV (SEQ ID NO:251); 4) KVDELAHFL (SEQ ID NO:252); 5) ALAETSYVKV (SEQ ID NO:253); and 6) ALSNKVDEL (SEQ ID NO:254).

HLA-A*02:01 with an amino acid sequence of GVAGDVSAV (SEQ ID NO:255, non naturally present) was selected as a negative control.

S2. Peptide exchange

S2-1. 5 μl peptide stock solution was mixed with 120 μl PBS buffer to dilute the 10 mM peptide stock solution to 400 μM to obtain a diluted peptide which was stored on ice;
S2-2. 20 μl of diluted peptide prepared in step s2-1 and 20 μl of peptide flex-t ™ HLA-A *02:01 monomer UVX (200 μG/ml) were injected into a 96-well V-shaped plate and mixed by using a pipette;
S2-3. the plate was sealed, and centrifuged at 2500xg at 4°C for 2min to obtain a supernatant;
S2-4. the plate was unsealed, placed on the ice and irradiated with an ultraviolet lamp for 30min, wit a distance between the ultraviolet lamp and the plate of 4cm; and
S2-5. the plate was sealed again, and cultured in the dark at 37°C for 30min to collect a liquid, which is s peptide exchange monomer.

In order to evaluate the efficiency of peptide exchange, a protocol of HLA class I ELISA was followed.

S3. Preparation of tetramer

S3-1. 30 μl of the peptide exchange monomer obtained in step S2 was transferred to 1.5ml micro centrifuge tube or new plate, then added with 3.3 μl of PE streptavidin (purchased from Biolegend under article No. 405204), mixed with a pipette, and incubated in the dark on ice for 30min;
S3-2. during incubation in step S3-1, 1.6 μl of 50 mM D-biotin and 6 μl of 10% (w/v) $NaN_3$ were added to 192.4 μl PBS buffer to prepare a blocking solution, 2.4 μl of which was added to the solution incubated in step S3-1 under vortex mixing, and mixed with a pipette to stop the reaction; and
S3-3. the micro centrifuge tube or plate was incubated at 5 °C overnight to obtain tetramer.

S4. Preparation of target group:

S4-1. before dyeing, the tetramer obtained in step S3 was centrifuged in a micro centrifuge tube or plate under 2500xg at 4 °C for 5min, and then stored on ice in the dark;

S4-2. 2*10$^6$ cells were added to 12*75mm tube or 96-well U-shaped base plate. The volume was adjusted to 200 $\mu$l with cell staining buffer. Each flex-t™ sample was added with 2 $\mu$l tetramer obtained in s4-1, mixed and cultured in the dark on ice for 30min;

S4-3. the cells were washed with the staining buffer twice and recultured in the staining buffer; and

S4-4. positive cell samples were collected by using a flow cytometry within 2 hours.

(3) Digesting of a patient's tumor tissue into a single cell suspension

After removing connective tissues from a surface of a tumor issue, the tumor issue was cut into 1cm* 1cm tissue pieces, fully shredded with a scissor, placed into a 15ml centrifuge tube. Each tube was added with 6 ml digestive solution of Miltenyi gentleMACS discociator KIT or a mixed solution of self-developed tissue digestive enzyme to digest the tissue.

Cell quality detection: Bio-Rad TC20 automatic cell counter was used in combination with a microscope to accurately detect the quality of the cell suspension, so as to ensure an experimental cell activity of greater than 70% and a cell diameter of less than 40% $\mu$m.

(4) Obtaining of TCR V(d)J full-length sequence capable of recognizing MAGE-A4 peptide

The TCR V(d)J full-length sequence capable of recognizing the MAGE-A4 peptide segment was obtained from the above four patients' tumors (2 MAGE-A4 positive esophageal cancer tissues, 1 MAGE-A4 positive bladder cancer tissue, and 1 MAGE-A4 positive head and neck tumor tissue).

A total of 30 TCR V(d)J full-length sequences were obtained, numbered TCR01 to TCR30. Each of the TCR V(d)J full-length sequences contains one TCRα polypeptide and one TCRβ polypeptide, the sequence of which is shown in Table 5.

Table 5

| TCR | TCRα polypeptide chain | TCRβ polypeptide chain |
|---|---|---|
| TCR01 | SEQ ID NO:1 | SEQ ID NO:31 |
| TCR02 | SEQ ID NO:2 | SEQ ID NO:32 |
| TCR03 | SEQ ID NO:3 | SEQ ID NO:33 |
| TCR04 | SEQ ID NO:4 | SEQ ID NO:34 |
| TCR05 | SEQ ID NO:5 | SEQ ID NO:35 |
| TCR06 | SEQ ID NO:6 | SEQ ID NO:36 |
| TCR07 | SEQ ID NO:7 | SEQ ID NO:37 |
| TCR08 | SEQ ID NO:8 | SEQ ID NO:38 |
| TCR09 | SEQ ID NO:9 | SEQ ID NO:39 |
| TCR10 | SEQ ID NO:10 | SEQ ID NO:40 |
| TCR11 | SEQ ID NO:11 | SEQ ID NO:41 |
| TCR12 | SEQ ID NO:12 | SEQ ID NO:42 |
| TCR13 | SEQ ID NO:13 | SEQ ID NO:43 |
| TCR14 | SEQ ID NO:14 | SEQ ID NO:44 |
| TCR15 | SEQ ID NO:15 | SEQ ID NO:45 |
| TCR16 | SEQ ID NO:16 | SEQ ID NO:46 |
| TCR17 | SEQ ID NO:17 | SEQ ID NO:47 |

(continued)

| TCR | TCRα polypeptide chain | TCRβ polypeptide chain |
|---|---|---|
| TCR18 | SEQ ID NO:18 | SEQ ID NO:48 |
| TCR19 | SEQ ID NO:19 | SEQ ID NO:49 |
| TCR20 | SEQ ID NO:20 | SEQ ID NO:50 |
| TCR21 | SEQ ID NO:21 | SEQ ID NO:51 |
| TCR22 | SEQ ID NO:22 | SEQ ID NO:52 |
| TCR23 | SEQ ID NO:23 | SEQ ID NO:53 |
| TCR24 | SEQ ID NO:24 | SEQ ID NO:54 |
| TCR25 | SEQ ID NO:25 | SEQ ID NO:55 |
| TCR26 | SEQ ID NO:26 | SEQ ID NO:56 |
| TCR27 | SEQ ID NO:27 | SEQ ID NO:57 |
| TCR28 | SEQ ID NO:28 | SEQ ID NO:58 |
| TCR29 | SEQ ID NO:29 | SEQ ID NO:59 |
| TCR30 | SEQ ID NO:30 | SEQ ID NO:60 |

**Example 2**

The difference of this example from Example 1 lies that, a different method for confirming HLA-A*02:01 typing in step (2) is adopted. In particular, the method for confirming HLA-A*02:01 typing in this example was as follows:
The sample was freeze stored as PBMC in cell cryopreservation solution. After thawing, the sample was washed twice with PBS to remove the cell cryopreservation solution, and centrifuged to remove the PBS buffer. RNA was extracted from obtained cell precipitate using Qiagen total RNA extraction kit. The RNA was subjected to denaturation, and reversely transcribed using Vazyme HiScript to obtain cDNA.

The cDNA was target enriched twice using Abclonal Gloria HS PCR Kit and corresponding primers of HAL1 and HLA2. 50 ng of the enriched product was subjected to fragmentation, terminal repair, linker connection and Index PCR amplification to construct a sequencing library using Abclonal FS DNA Lib Prep Kit.

The library was subjected to quality inspection and then sequencing on Illumina Novaseq6000 sequencing platform. A target data volume of each sample was 9G.

**Example 3 Construction of plasmid, virus and cells from TCR-T**

(1) Construction of plasmid

A complete TCR sequence (TRA V(d)J + C domain linking α chain + linker domain + TRB V(d)J + C domain linking β chain) was cloned into a target plasmid of lentivirus system (for example, in PGK), and transfected into E. coli. Positive clones with kanamycin resistance were selected for amplification and culture, and plasmid was extracted by using Qiagen Plasma Maxi Kit.

The linker domain was modified P2A (SEQ ID No: 241) or P2A linker sequence (SEQ ID No: 242).

In this example, C domain sequences selected for TCR01-TCR05 were SEQ ID No: 243 and SEQ ID No: 244, and the linker domain was modified P2A (SEQ ID No: 241);

C domain sequences selected for TCR06-TCR10 were SEQ ID No: 245 and SEQ ID No: 246, and the linker domain was modified P2A (SEQ ID No: 241);
C domain sequences selected for TCR11-TCR15 were SEQ ID No: 243 and SEQ ID No: 244, and the linker domain was P2A linker sequence (SEQ ID No: 242);
C domain sequences selected for TCR15-TCR20 were SEQ ID No: 245 and SEQ ID No: 246, and the linker domain was P2A linker sequence (SEQ ID No: 242);
C domain sequences selected for TCR16-TCR20 were SEQ ID No: 247 and SEQ ID No: 248, and the linker domain was modified P2A (SEQ ID No: 241);
C domain sequences selected for TCR21-TCR25 were SEQ ID No: 243 and SEQ ID No: 244, and the linker domain was modified P2A (SEQ ID No: 241); and

C domain sequences selected for TCR26-TCR30 were SEQ ID No: 243 and SEQ ID No: 244, and the linker domain was C linker sequence (SEQ ID No: 242).

(2) Virus packaging

1) 24h before packaging, 8*10[6] 293T cells were spread out in a 10cm Petri dish, supplemented with 10 ml DMEM medium containing 10% FBS, and then cultured in a 5% $CO_2$ incubator at 37 °C;
2) the cells were observed on the day of packaging, and lentivirus packaging was performed when it was confirmed that the cells reached a confluence of 80% ($\pm$2%) and assumed a transparent state;
3) reagents used during packaging were removed and balanced to room temperature;
4) 10 ml of the medium in Petri dish and 9ml serum-free DMEM medium added along the dish wall were transfer to $CO_2$ incubator for use;
5) 450 $\mu$l of Opti-MEM was added to a 1.5 ml centrifuge tube, and then added with 7.5 $\mu$g of psPAX2, 5 $\mu$g of pMD2. G and 10 $\mu$g of target plasmid, and mixed with a pipette;
6) another 1.5 ml centrifuge tube was add with 450 $\mu$l of Opti-MEM and 22.5 ul of PEIpro reagent, and mixed with a pipette;
7) a mixture obtained in step 5) with a mixture obtained in step 6) were mixed with a pipette, and incubated at room temperature for 10-15 min;
8) a mixture obtained in step 7) was gently dripped into a 10cm Petri dish, and then transferred to a $CO_2$ incubator for further culturing;
9) after 6h, the medium in the 10cm culture dish obtained in step 8) was removed, 10 ml of DMEM medium containing 10% FBS was added to the dish along the dish wall, and the dish was transferred to $CO_2$ incubator for further culturing;
10) After culturing the medium obtained in step 9) for 48h, supernatant was collected into a 50 ml centrifuge tube, 10 ml of DMEM medium containing 10% FBS was added along the dish wall, the dish was transferred to $CO_2$ incubator for further culturing, and the supernatant containing virus was stored in a refrigerator at 4 °C;
11) after 72h, the supernatant stored in step 10) was collected into a 50ml centrifuge tube, and centrifuged at 500 $\times$ g and 4 °C for 10 min to remove cell debris;
12) the supernatant obtained upon centrifuging in step 11) was filtered through 0.45 $\mu$m filter into a new 50ml centrifugal tube;
13) the supernatant obtained upon filtering in step 12) was added with a quarter volume of PEG8000 concentration reagent and incubated in a shaking table at 4 °C for more than 3h;
14) the virus solution incubated in step 12) was centrifuged at 2000$\times$g and 4 °C for 40 min to obtain a precipitate, which was added with PBS precooled at 4 °C, and gently mixed with a pipette; and
15) The virus obtained in step 14) is divided an packaged into cryopreservation tubes and stored at -80 °C for use.

The virus obtained in the above steps was subjected to virus titration test as follows:
1) on the day of test, the cell density of Jurkat was adjusted to 3*10[5] cells/ml, added to a 24-well plate by 1 ml cell suspension/each well, and then cultured in 5% $CO_2$ incubator at 37 °C for use;
2) the virus was gradient diluted according to the ratios listed in Table 6 below, in which an additional group of secondary wells was provided for each group;

Table 6

| Diluting ratio | Volume of virus ($\mu$L) | Volume of 1640 medium ($\mu$L) | 10mg/mL polybrene($\mu$L) |
| --- | --- | --- | --- |
| 1:50 | 30 | 468.5 | 1.5 |
| 1:100 | 15 | 483.5 | 1.5 |
| 1:200 | 7.5 | 483.5 | 1.5 |
| 1:400 | 3.75 | 494.75 | 1.5 |

3) the diluted virus solution was added to cells, centrifuged at 800g and 21 °C for 2h, and then cultured in 5% $CO_2$ incubator at 37 °C;
4) after 60 h, the cells were subjected to FACS test.
Titer was calculated by:

$$Tu/ml = ((\text{number of infected cells}) \times \text{positive rate} \times (\text{times of dilution}))/\text{infected volume}$$

For example, when the positive rate of one well with a dilution ratio of 1:100 is 25%, the titer is $(3*10^5) \times 0.25 \times 100)/(1.5ml) = 5*10^6$ TU/ml

When calculating the titer, only the wells with a positive rate of less than 40% are considered, for the reason that, when the positive rate is higher than 40%, the possibility of multiple virus particles repeatedly infecting one cell will result in inaccurate titer calculation.

(3) Thawing of PBMC:

1) 30 ml of L500 medium was added into 50 ml centrifuge tube and restored to room temperature for use;
2) frozen PBMC was removed from -80 °C refrigerator, quickly placed into a 37 °C water bath, and quickly shaked until there was no visible ice in the frozen tube;
3) thawed cryopreservation tube was disinfected with 75% alcohol, transferred to a biosafety cabinet, slowly dripped into the thawed L500 medium with a 1 ml pipette, and centrifuged at 400g and 25 °C for 5min;
4) supernatant was discarded after centrifugation, the cells were resuspended in 30ml of pre-heated L500 medium, from which 10 ul of the sample was taken for living cell counting, followed by centrifuging at 25 °C and 400g for 5min.

(4) Stimulation and activation of PBMC

1) based on counting results, cells were resuspended in L500 medium and the density of living cells was adjusted to $1-2e^6$/ml;
2) a suspension obtained in step 1) was added with 300IU/ml IL-2 and 50 ng/ml CD3 antibody, mixed, and transferred to $CO_2$ incubator where they were stimulated and activated for 60 h.

(5) Lentivirus transduction

1) activation of PBMC 48 hours after activation was observed, and lentivirus transduction was carried out;
2) virus was added by MOI = 2 and cells were transferred into carbon dioxide incubator and cultured for 48 h;
3) the cells were transferred into a 50ml centrifuge tube from which 10 $\mu$l was taken for living cell counting, centrifuged at 25 °C and 400 g for 5min, and washed;
4) based on the results of counting, the transfected cells were resuspended in L500 medium, the cell concentration was adjusted to $1e^6$/ml, IL-2 was added by 100IU/ml, and the cells were transferred to $CO_2$ incubator for culturing.

(6) Expanding and culturing of cells

1) after lentivirus transduction and solution replacing, cells were observed and counted every day, and controlled to have a number of $1e^6$/ml and IL-2 concentration of 100IU/ml by supplementing medium; and
2) the cells were harvested on the ninth day of culturing.

**Example 4 cell line killing experiment**

Positive: A375, h1755, h1395 - (MAGE-A4+, HLA-A02+)
Negative: H1299 - (MAGE-A4-, HLA-A02-)

T cells screened in this application that can specifically recognize MAGE-A4 positive tumors and target cells, by a ratio of 5:1, were subjected to relevant experiments. The results are shown in FIG. 2, where the abscissa 1-30 represents TCR01-TCR30, respectively, and the ordinate represents a percentage of killing.

The T cells prepared using TCR01-TCR30 were mixed with the target cells with positive A375, H1755 and H1395 respectively by a ratio of T cells to target cells of 5:1. The results are shown in FIG 2, where the abscissa 1-30 represents TCR01-TCR30, respectively, and the ordinate represents the percentage of killing.

The results show that, TCR-T cells constructed using TCRs screened in this application have the most significant killing effect on target cells with positive H1755 and H1395, with a cell killing percentage of 70%-90%. TCR-T cells constructed using TCRs screened in this application have a relatively significant killing effect on cells with positive A375, with a cell killing percentage of 50%-70%. The killing effect of T cells screened in this application on cells with H1299 showing HLA-A*02:01 negative and having MAGE-A4 knockout is very low, that is, less than 10%.

**Example 5 Tumor clearance experiment in mice**

NCG mice were inoculated with $1e^7$ A375, H1395, H1755 and H1395 (MAGE-A4-), in which the first three cell lines were natural HLA-A*02:01 and MAGE-A4 positive, and H1395 (MAGE-A4-) has MAGE-A4 knocking-out. When the tumor grew to a volume of 100 mm$^3$, the mice were infused with 200 $\mu$l suspension containing $3*10^6$ TCR-T cells at caudal vein for one time, and then subjected to tumor size measurement and weight detection. FIG. 3A-FIG. 3D show the results of tumor clearance, in which the abscissa represents days from medication and the ordinate represents a change in tumor size with the increase of days from medication.

FIG. 3A shows that, in the NCG H1395 tumor mice having an initial tumor size of about 100 mm$^3$, there is a small increase in 1-5 days after TCR-T injection, that is, the tumor size being increased to 200 mm$^3$; and during 5-18 days, the tumor size is decreased significantly. The tumor size in the control group MOCK is increased to 800 mm$^3$ on the 18th day, decreased to 100 mm$^3$ or below after injection treatment, and after TCR05 injection, remains at about 100 mm$^3$. The tumor size of other cells after TCR-T injection was decreased significantly, approaching 0.

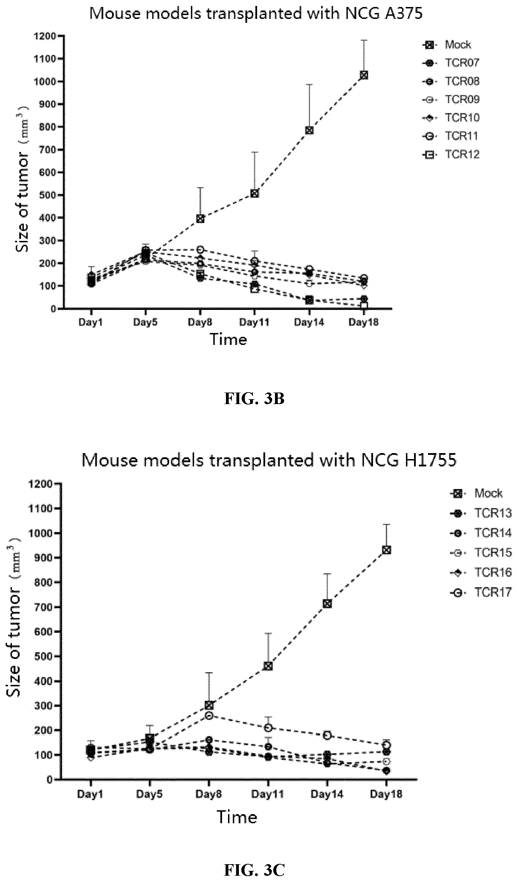

FIG. 3B shows that, in the NCG A375tumor mice having an initial tumor size of about 100 mm$^3$, there is a small increase in 1-8 days after TCR-T injection, that is, the tumor size being increased to 250 mm$^3$; and during 8-18 days, the tumor size is decreased significantly. The tumor size in the control group MOCK is increased to 1000 mm$^3$ or above on the 18th day, and decreased to 100 mm$^3$ or below after injection treatment. The tumor size of other cells after TCR07 and TCR12 injection approached 0, showing a good result.

FIG. 3C shows that, in the NCG H1755 tumor mice having an initial tumor size of about 100 mm$^3$, the tumor size in the control group MOCK is increased to 900 mm$^3$ during 1-18 days. In the TCR-T treatment group, the tumor size fluctuates greatly, except for TCR17 group. In other groups, the tumor size fluctuates gently after TCR-T cell injection, and an overall volume of cells is not increased within 18 days and remains at or below 100 mm$^3$.

FIG. 3D shows that, in the tumor mice of H1395 (MAGE-A4- and HLA-A02+) with MAGE-A4 knockout or wild-type H1395 (MAGE-A4+ and HLA-A02+) tumor mice having an initial tumor size of about 100 mm$^3$, treatment of TCR01-05-T cells mixture are significantly impaired when the tumor cells assume MAGE-A4 negative, and the tumor size reaches 800-900 mm$^3$ 18 days after injection. When the tumor cells assume MAGE-A4 positive, injection of TCR-T cells according to the present application can significantly inhibit the increase of cell volume, which is controlled to be 100 mm$^3$ or below on the 18th day from injection.

FIG. 3E shows a weight change of mice after injection. It can be seen that, there is no significant change in the weight of mice during the whole injection period, indicating that TCR-T cells according to the present application will not cause obvious toxic reaction after injection.

The results from FIG. 3a to FIG. 3E show that, the constructed TCR-T cells have good tumor inhibition effect and low toxicity in vivo.

**Example 6 IFN ELISA test confirming no reaction with normal cell lines**

TCR01-TCR15 were selected to react with 11 kinds of normal tissue cells and positive h1755 (MAGE-A4+ and HLA-A02+) cells. INF content in individual cells were detected after 3 days. The selected normal tissue cells are shown in Table 7 below:

Table 7

| Chinese name of primary cells | Article No. |
|---|---|
| Pulmonary type II alveolar epithelial cells | CTCC-A005-PC |
| Cardiomyocyte | CTCC-C002-PC |

(continued)

| Chinese name of primary cells | Article No. |
|---|---|
| Esophageal epithelial cells | CTCC-D001-PC |
| Gastric mucosal epithelial cells | CTCC-D004-PC |
| Intestinal mucosal epithelial cells | CTCC-D007-PC |
| Colonic mucosal epithelial cells | CTCC-D011-PC |
| Gallbladder epithelial cells | CTCC-D014-PC |
| Hepatic parenchymal cells | CTCC-D017-PC |
| Renal tubular epithelial cells | CTCC-U002-PC |
| Bladder epithelial cells | CTCC-U008-PC |
| Islet cells | CTCC-G004-PC |

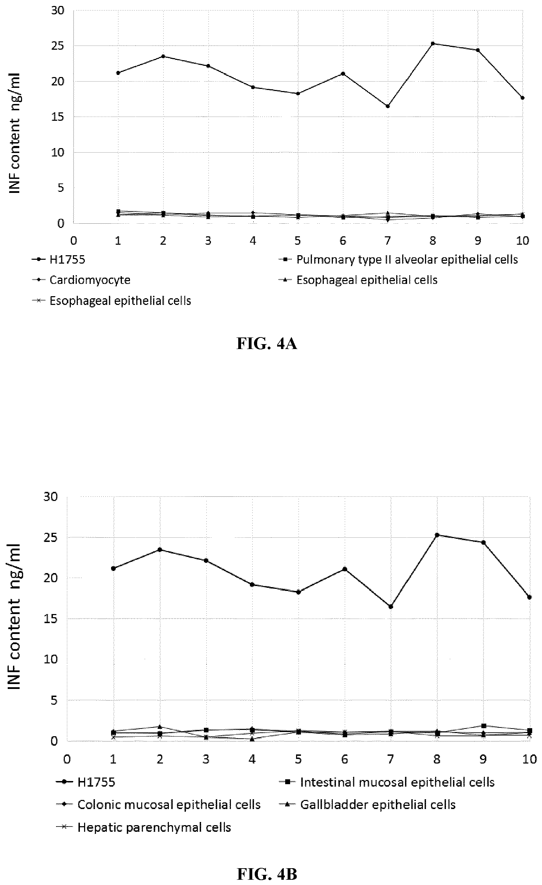

The test results are shown in FIG. 4A-FIG. 4C, in which the abscissa is TCR01-TCR10 and the ordinate is INF content. In FIG. 4A, pulmonary type II alveolar epithelial cells, cardiomyocytes, esophageal epithelial cells and gastric mucosal epithelial cells are illustrated; and in FIG. 4B, small intestinal mucosal epithelial cells, colonic mucosal epithelial cells, gallbladder epithelial cells and hepatic parenchymal cells are illustrated; and in FIG. 4C, renal tubular epithelial cells and bladder epithelial cells are illustrated.

When TCR-T cells according to the present application are injected, INF content in 11 normal tissue cells is maintained at 0-5ng/ml, approaching 0 ng/ml, indicating that TCR-T cells are basically non-toxic to normal cells, while in positive H1755 (MAGE-A4+, HLA-A02+) tumor cells, the INF content can reach up to 15-255 ng/ml.

**Example 7 Effect of TCR-T screened in this application in treating tumor patients**

Patient PA2021-TCR01, male, 75 years old, was diagnosed with high-grade papillary urothelial carcinoma of the bladder in September 2020 due to gross hematuria. After performing relevant examinations, bladder space occupation was considered. Transurethral resection of bladder tumor was performed in October 2020. Postoperative pathology showed that high-grade papillary urothelial carcinoma of the bladder infiltrated the muscular layer. PET CT showed positive FDG uptake in multiple pelvic lymph nodes, and metastasis was considered. After diagnosis, the patients were treated with gemcitabine + cisplatin + teplizumab for 4 times. The tumor still progressed after reexamination in March 2021. After being treated with MAGE-A4 TCR-T cells, IHC staining result of tumor pathological white film showed MAGE-A4 positive (FIG. 5), and the HLA typing result of peripheral blood was HLA-A*02:01, as shown in Table 8 below.

Table 8

| PA2021-TCR01_HLA | PA2021-TCR01_Read_counts | PA2021-TCR01_HLA_cell_num | PA2021-TCR01_Total_cell_num | PA2021-TCR01_Cell_r atio |
|---|---|---|---|---|
| A*02:01:175 | 28510 | 2703 | 2983 | 0.9061 |
| B*08:18 | 31832 | 2671 | 2983 | 0.8954 |
| C*03:47 | 2300 | 1287 | 2983 | 0.4314 |
| C*07:429 | 10576 | 2289 | 2983 | 0.7673 |

TCR01-05 cell preparation containing $5e^9$ TCR-T cells were infused intravenously. MR reexamination results on the day of injection (FIG. 6A) and 45 days after injection (FIG. 6B) showed that the focus was reduced and the diffusion was weakened, and the maximum diameter of the tumor was reduced from 48.52 mm to 34.48 mm.

The above examples are only provided for the purpose of explaining the technical concept and characteristics of the application, so as to enable those skilled in the art familiar with the technology and understand the content of the application and implement it. They are not intended to limit the scope of protection of the application. Without departing from the concept, spirit and scope of the application, the method described in the application and the steps or step sequence of the method can be changed. All equivalent changes or modifications made according to the spirit and essence of this application shall be covered by the scope of protection of this application.

**Claims**

1. A polypeptide comprising a TCRα polypeptide having CDRα1 consisting of SEQ ID No: 64, CDRα2 consisting of SEQ ID No: 94 and CDRα3 consisting of SEQ ID No: 124; and a TCRβ polypeptide having CDRβ1 consisting of SEQ ID No: 154, CDRβ2 consisting of SEQ ID No: 184 and CDRβ3 consisting of SEQ ID No: 214.

2. The polypeptide according to claim 1, wherein the polypeptide comprises a TCRα polypeptide having at least 90% identity with the amino acid sequences of SEQ ID No: 4 and a TCRβ polypeptide having at least 90% identity with the amino acid sequences of SEQ ID No: 34.

3. The polypeptide according to claim 1, wherein the polypeptide comprises a TCRα polypeptide having at least 95% identity with the amino acid sequences of SEQ ID No: 4 and a TCRβ polypeptide having at least 95% identity with the amino acid sequences of SEQ ID No: 34.

4. The polypeptide according to claim 1, wherein the polypeptide comprises a TCRα polypeptide of SEQ ID No: 4 and/or a TCRβ polypeptide of SEQ ID No: 34.

5. A T cell receptor (TCR) capable of specifically recognizing MAGE-A4 antigenic peptide, comprising a TCRα Polypeptide having at least 90% identity with the sequence of SEQ ID No: 4 and a TCRβ polypeptide having at least 90% identity with the sequence of SEQ ID No: 34.

6. The TCR according to claim 5, wherein the sequence of SEQ ID No: 4 contains CDRα1 (SEQ ID No: 64), CDRα2 (SEQ ID No: 94) and CDRα3 (SEQ ID No: 124); and the sequence of SEQ ID No: 34 contains CDRβ1 (SEQ ID No: 154), CDRβ2 (SEQ ID No: 184) and CDRβ3 (SEQ ID No: 214).

7. The TCR according to claim 5, wherein the TCRα polypeptide has at least 95% identity with the sequence of SEQ ID No: 4, and the TCR β polypeptide has at least 95% identity with the sequence of SEQ ID No: 34.

8. The TCR according to claim 5, wherein the TCRα polypeptide has at least 99% identity with the sequence of SEQ ID No: 4, and the TCRβ polypeptide has at least 99% identity with the sequence of SEQ ID No: 34.

9. The TCR according to any of claims 1-8, wherein the antigenic peptide is HLA-A2 restricted.

10. The TCR according to claim 9, wherein the HLA-A2 is HLA-A*0201 typing.

11. A polynucleotide encoding the polypeptide according to any of claims 1-4 or the TCR according to any of claims 5-10.

12. An expression vector containing a polynucleotide encoding the TCR according to any of claims 1-4.

13. A host cell comprising the polynucleotide of claim 12 for expression of the polypeptide according to any of claims 1-4 or the TCR according to any of claims 5-10, preferably the host cell is immune cell, more preferably the immune cell is selected from T cells, NK cells, constant NK cells, NKT cell and other lymphocytes in peripheral blood.

14. A composition comprising an effective therapeutic dose of MAGE-A4 TCR specific cells for use in the treatment of a cancer, optionally, the caner is selected from a group consisting of esophageal cancer, gastric cancer, bladder cancer, head and neck tumor or sarcoma.

**FIG. 1A**

**FIG. 1B**

**FIG. 1C**

**FIG. 1D**

**FIG. 2**

**FIG. 3A**

Mouse models transplanted with NCG A375

FIG. 3B

Mouse models transplanted with NCG H1755

FIG. 3C

**FIG. 3D**

**FIG. 3E**

**FIG. 4A**

**FIG. 4B**

**FIG. 4C**

**FIG. 5**

**FIG. 6A**

**FIG. 6B**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/257288 A2 (REGENERON PHARMA [US]) 24 December 2020 (2020-12-24) | 14 | INV. C07K14/725 A61K38/00 |
| A | * abstract; claims 23-25 * | 1-13 | |
| A | WO 2022/235577 A2 (UNIV PITTSBURGH COMMONWEALTH SYS HIGHER EDUCATION [US] ET AL.) 10 November 2022 (2022-11-10) * TCR alpha comprising SEQ ID NOs:64 and 164 of the current application; sequence 371 * | 1-14 | |
| A | WO 2022/118030 A1 (UNIV OXFORD INNOVATION LTD [GB]) 9 June 2022 (2022-06-09) * TCR alpha comprising SEQ ID NOs:64 and 164 of the current application; claim 7; sequence 80 * | 1-14 | |
| A | WO 2016/146618 A1 (MAX-DELBRÜCK-CENTRUM FÜR MOLEKULARE MEDIZIN [DE] ET AL.) 22 September 2016 (2016-09-22) * TCR beta comprising SEQ ID NOs:154 and 184 of the current application; claim 5; sequence 16 * | 1-14 | TECHNICAL FIELDS SEARCHED (IPC) C07K A61K |
| A | WO 2022/040569 A1 (US HEALTH [US]; VIZCARDO RAUL E [US] ET AL.) 24 February 2022 (2022-02-24) * TCR beta comprising SEQ ID NOs:154 and 184 of the current application; claim 81; sequence 14 * | 1-14 | |

The present search report has been drawn up for all claims

| Place of search | Date of completion of the search | Examiner |
|---|---|---|
| Munich | 8 May 2024 | Luo, Xinmei |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 16 2117

This annex lists the patent family members relating to the patent documents cited in the above-mentioned European search report.
The members are as contained in the European Patent Office EDP file on
The European Patent Office is in no way liable for these particulars which are merely given for the purpose of information.

08-05-2024

| Patent document cited in search report | | Publication date | Patent family member(s) | | Publication date |
|---|---|---|---|---|---|
| WO 2020257288 | A2 | 24-12-2020 | AU | 2020295401 A1 | 10-02-2022 |
| | | | BR | 112021025548 A2 | 17-05-2022 |
| | | | CA | 3144253 A1 | 24-12-2020 |
| | | | CL | 2021003366 A1 | 19-08-2022 |
| | | | CN | 114585646 A | 03-06-2022 |
| | | | CO | 2022000281 A2 | 28-01-2022 |
| | | | EP | 3986938 A2 | 27-04-2022 |
| | | | IL | 289042 A | 01-02-2022 |
| | | | JP | 2022537359 A | 25-08-2022 |
| | | | KR | 20220035387 A | 22-03-2022 |
| | | | MA | 56548 A | 27-04-2022 |
| | | | US | 2022324939 A1 | 13-10-2022 |
| | | | WO | 2020257288 A2 | 24-12-2020 |
| WO 2022235577 | A2 | 10-11-2022 | AU | 2022271195 A1 | 02-11-2023 |
| | | | CA | 3217621 A1 | 10-11-2022 |
| | | | EP | 4334339 A2 | 13-03-2024 |
| | | | IL | 308090 A | 01-12-2023 |
| | | | JP | 2024518403 A | 01-05-2024 |
| | | | KR | 20240026910 A | 29-02-2024 |
| | | | WO | 2022235577 A2 | 10-11-2022 |
| WO 2022118030 | A1 | 09-06-2022 | EP | 4255572 A1 | 11-10-2023 |
| | | | US | 2024024477 A1 | 25-01-2024 |
| | | | WO | 2022118030 A1 | 09-06-2022 |
| WO 2016146618 | A1 | 22-09-2016 | AU | 2016232280 A1 | 12-10-2017 |
| | | | CA | 2979493 A1 | 22-09-2016 |
| | | | CN | 107636152 A | 26-01-2018 |
| | | | DK | 3271382 T3 | 04-05-2020 |
| | | | EP | 3271382 A1 | 24-01-2018 |
| | | | ES | 2790725 T3 | 29-10-2020 |
| | | | HK | 1248736 A1 | 19-10-2018 |
| | | | JP | 6944876 B2 | 06-10-2021 |
| | | | JP | 2018509911 A | 12-04-2018 |
| | | | KR | 20170126500 A | 17-11-2017 |
| | | | SG | 11201707540Q A | 30-10-2017 |
| | | | US | 2018073013 A1 | 15-03-2018 |
| | | | US | 2021261947 A1 | 26-08-2021 |
| | | | WO | 2016146618 A1 | 22-09-2016 |
| WO 2022040569 | A1 | 24-02-2022 | EP | 4200407 A1 | 28-06-2023 |
| | | | JP | 2023538418 A | 07-09-2023 |
| | | | US | 2023265508 A1 | 24-08-2023 |
| | | | WO | 2022040569 A1 | 24-02-2022 |

EPO FORM P0459

For more details about this annex : see Official Journal of the European Patent Office, No. 12/82